# FASCICULE DE BREVET EUROPEEN

(11) **EP 3 183 284 B1**
(45) Date de publication et mention de la délivrance du brevet: **18.07.2018**
(21) Numéro de dépôt: 15748188.8
(22) Date de dépôt: 21.07.2015
(51) Int. Cl.: C08G 59/34, C08G 59/02, C07H 13/06, C07H 15/203

(54) **DERIVES POLYESTERS D'ACIDES GRAS DE POLYGLYCOSIDES**
FETTSÄUREPOLYESTERDERIVATE VON POLYGLYCOSIDEN
FATTY ACID POLYESTER DERIVATIVES OF POLYGLYCOSIDES

(30) Priorité: 22.08.2014 FR 1457946
(43) Date de publication de la demande: 28.06.2017
(73) Titulaire: Université de Montpellier, 34090 Montpellier (FR); Centre National de la Recherche Scientifique, 75016 Paris (FR)
(72) Inventeur: PATRY, Stéphane, F-38500 Voiron (FR); HABAS, Jean-Pierre, F-34000 Montpellier (FR)
(74) Mandataire: Pontet Allano & Associes
(86) Numéro de dépôt international: PCT/EP2015/066690
(87) Numéro de publication internationale: WO 2016/026641

(56) Documents cités:
- EP-A1- 0 132 941
- WO-A1-2011/097484
- US-A1- 2006 020 062

## Description

La présente invention a pour objet des dérivés polyesters d'acides gras de polyglycosides biosourcés, leur procédé de préparation et leur utilisation comme synthons dans la préparation de différents matériaux.

Les polymères synthétiques font partie de l'univers quotidien. Légers, résistants, isolants, faciles à mettre en oeuvre, ils sont utilisés dans les secteurs aussi divers que le bâtiment, l'emballage, l'industrie électrique et électronique, l'électroménager, le secteur du jouet et les transports (automobile, ferroviaire, aéronautique...). Le caractère fossile et limité des ressources pétrolières et les considérations relatives à la qualité de l'environnement (réchauffement climatique, gestion des déchets) offrent une réelle opportunité aux polymères biosourcés à savoir issus non plus du pétrole mais de la biomasse.

Parmi ces polymères, les résines époxydes, par leurs propriétés physicochimiques (mécaniques, électriques,...) constituent une classe polyvalente de polymères thermodurcissables très largement utilisée dans les domaines de l'électronique, du bâtiment, des peintures ou encore des transports. La très grande majorité de celles qui sont actuellement commercialisées sont d'origine pétrochimique. Elles sont formées par le mélange d'un (ou plusieurs) « prépolymère(s) époxy » (autrement dit d'une molécule porteuse de groupements réactifs époxy) avec un durcisseur également désigné sous le terme « d'agent de réticulation ». Ces deux familles de composés (prépolymère et durcisseur) réagissent entre eux par polymérisation qui, suivant la fonctionnalité des espèces chimiques mises en jeu, peut engendrer la production d'un réseau polymère tridimensionnel. Les résines époxy sont alors dites réticulées.

De nombreuses formulations époxy font appel à un prépolymère pétrosourcé, le diglycidyl éther de bisphénol A (ou DGEBA). Or, ce dernier est formulé à partir de bisphénol A qui est un composé classé CMR (cancérogène, mutagène, reprotoxique). Son époxydation est elle-même critiquable sur le plan sanitaire car elle recourt à de l'épichlorhydrine dont la toxicité chimique fait aujourd'hui débat et dont l'usage risque à moyen-terme d'être lui-aussi restreint voir supprimé.

Ainsi, pour trouver une réponse à des contraintes règlementaires de plus en plus exigeantes (REACH, RoHS...), mais aussi pour faire face à l'épuisement inéluctable de la ressource pétrolière, diverses recherches ont été menées pour tenter de développer des matériaux, notamment des polymères, en particulier des résines époxydes, issus de la biomasse.

Ainsi les inventeurs ont décrit dans la demande WO 2013/124574 des résines époxydes comprenant le produit de réaction d'un ou de plusieurs dérivés lipidiques époxydés biosourcés extraits d'une huile végétale naturelle, avec au moins un agent de réticulation, en présence d'au moins un co-réactif choisi parmi les dérivés glycidyl éthers de polyols biosourcés.

Par la suite, les Inventeurs ont d'une part cherché à accroitre encore les performances de ces résines biosourcées, notamment sur le plan de la température de transition vitreuse et d'autre part à développer une approche polyvalente permettant la création de molécules plateformes dotées de caractéristiques chimiques (nature et nombre de fonctionnalités) permettant un large éventail de déclinaisons (polyépoxy, polyamine, polyols...) donc d'applications potentielles.

Le brevet français FR2962131 décrit des corps gras d'origine naturelle fonctionnalisés par des fonctions alcools primaires ou amines primaires par une réaction de type thiol-ène et leur utilisation pour préparer, par polycondensation, des polymères de différents types : polyesters, polyamides, polyuréthanes, résines époxy.

Le brevet US6995232 décrit la synthèse de polyesters de saccharose (SEFOSE) par la réaction d'esters d'acide gras individuel sur du saccharose. La structure finale est ainsi formée par un coeur de type saccharidique (un cycle de glucose « naturellement » lié à un cycle de fructose) sur lequel sont greffées jusqu'à huit chaines grasses (SEFOSE).

La demande internationale WO 2011/097484 décrit la fonctionnalisation par époxydation des SEFOSE décrits par le brevet US6995232 et la demande internationale WO02/060975. Le concept chimique proposé ne concerne que la phase dite d'époxydation à proprement parler et n'autorise aucune modulation du coeur central des structures moléculaires initiales pourtant essentielle à une polyvalence de fonction et d'usage.

Pourtant, il existe encore un besoin de disposer de structures moléculaires polyvalentes afin de permettre la fabrication de matériaux partiellement ou totalement biosourcés et présentant une large gamme de caractéristiques mécaniques, notamment pour concurrencer celles des polymères d'origine pétrochimique et afin de s'adapter à de nombreuses fonctions d'usage final.

Or les Inventeurs ont découvert qu'en greffant des chaines grasses individuelles (notamment des acides gras issus d'huiles végétales ou esters d'acides gras issus d'huiles végétales) sur une structure biosourcée modulable car à base de sucres réducteurs liés entre eux par un segment de nature aliphatique, cycloaliphatique ou aromatique et qui permet de moduler la rigidité du coeur central, il était possible d'obtenir une large gamme de molécules en jouant soit sur la nature du coeur central, soit sur la fonctionnalisation des chaines grasses greffées. Chacune est dotée de propriétés spécifiques permettant ainsi de répondre à des fonctions précises et au final de satisfaire à une grande polyvalence d'applications potentielles.

Aussi la présente invention a pour objet des dérivés polyesters d'acides gras de polyglycosides formés à partir d'un polyol comprenant entre 2 et 10 fonctions hydroxy, avantageusement entre 2 et 5 fonctions hydroxy, les 2 fonctions hydroxy ou au moins 2 de ces fonctions hydroxy étant liées au carbone anomérique d'un glucide réducteur, identique ou différent pour chaque groupe hydroxy et choisi parmi les monosaccharides et les disaccharides, dans lesquels l'un au moins des autres hydroxy dudit monosaccharide ou dudit disaccharide est estérifié par un dérivé lipidique porteur d'au moins une double liaison et éventuellement issu d'une huile végétale ou animale ou d'un mélange d'huiles végétales ou animales, la double liaison ou l'une au moins des doubles liaisons dudit dérivé lipidique étant fonctionnalisée par un groupe choisi parmi les groupes époxy, amines, alcools et acides.

Conformément à l'invention, les fonctions hydroxy des mono- ou des disaccharides non estérifiées par des chaînes lipidiques insaturées (chaînes porteuses d'au moins une double liaison) peuvent être soit libres, soit estérifiées par une chaîne lipidique saturée en C₄-C₃₆, avantageusement en C₁₂-C₂₀.

Au sens de la présente invention, le terme «biosourcé» désigne un produit issu de la biomasse. La biomasse décrit la masse totale d'organismes vivants d'origine végétale ou animale dans un milieu défini, appelé biotope, et les ressources qui en découlent au travers d'une utilisation directe, indirecte ou potentielle pour l'humanité.

Dans un mode de réalisation avantageux de l'invention, les dérivés polyesters d'acides gras de polyglycosides répondent à la formule (I) 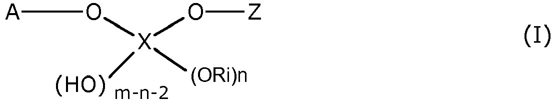 dans laquelle
- A et Z représentent chacun, indépendamment l'un de l'autre, un glucide réducteur choisi dans le groupe des monosaccharides comprenant le glucose, le fructose, le galactose et le mannose ou dans le groupe des disaccharides comprenant le lactose et le maltose,
   ∘ lesdits monosaccharides et lesdits disaccharides étant liés à -O-X-O- par leur carbone anomérique portant initialement le OH hémiacétalique,
   ∘ l'un au moins des autres OH dudit monosaccharide ou dudit disaccharide étant estérifié par un dérivé lipidique porteur d'au moins une double liaison éventuellement issu d'une huile végétale ou animale ou d'un mélange d'huiles végétales ou animales,
   ∘ la double liaison ou l'une au moins des doubles liaisons dudit dérivé lipidique étant fonctionnalisée par un groupe choisi parmi les groupes époxy, amines, alcools et acides,
- X représente une structure chimique porteuse des fonctions hydroxy dans un composé choisi dans le groupe comprenant les polyols aliphatiques (chaînes non cycliques), cycloaliphatiques et aromatiques,
- Rᵢ représente soit un substituant, soit plusieurs substituants notés Rₐ à Rₕ, lesdits substituants Rₐ à Rₕ, identiques ou différents, étant des glucides réducteurs choisis dans le groupe des monosaccharides comprenant le glucose, le fructose, le galactose et le mannose ou dans le groupe des disaccharides comprenant le lactose et le maltose,
   ∘ lesdits monosaccharides et lesdits disaccharides étant liés à-O-X-O- par leur carbone anomérique portant initialement le OH hémiacétalique,
   ∘ l'un au moins des autres OH dudit monosaccharide ou dudit disaccharide étant estérifié par un dérivé lipidique porteur d'au moins une double liaison éventuellement issu d'une huile végétale ou animale ou d'un mélange d'huiles végétales ou animales,
   ∘ la double liaison ou l'une au moins des doubles liaisons dudit dérivé lipidique étant fonctionnalisée par un groupe choisi parmi les groupes époxy, amines, alcools et acides,
- m correspondant au nombre d'hydroxyles du polyol dont X est issu est un entier compris entre 2 et 10 et
- n, nombre de glucides réducteurs additionnels, est inférieur ou égal à m-2.

Ainsi, conformément à l'invention, les composés sont soit des dérivés polyesters d'acides gras de biglycosides, de triglycosides, de tétraglucosides, de pentaglucosides, d'hexaglucosides, d'heptaglucosides, d'octaglucosides, de nonaglucosides ou de décaglucosides.

Avantageusement X est une structure chimique porteuse de groupements hydroxy provenant d'un polyol choisi parmi le glycérol, le xylitol, le phloroglucinol, l'érythritol, le pentaérythritol, le dipentaérythritol, l'arabitol, le ribitol, le sorbitol, le dulcitol, le mannitol, le volemitol, le maltitol, l'isomaltitol et le lactitol, ou d'un diol choisi parmi les diols répondant à l'une des formules suivantes :

| **Nom** | **Formule** |
|---|---|
| 1,3-propanediol | 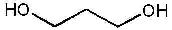 |
| 1,4-butanediol | 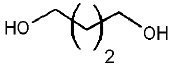 |
| 1,5-pentanediol | 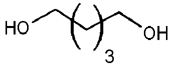 |
| 1,12-dodecanediol | 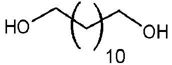 |
| diéthylène glycol | 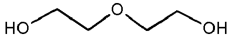 |
| pentaéthylène glycol | 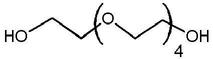 |
| 2-butène-1,4-diol | 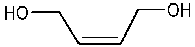 |
| 2-butyne-1,4-diol | 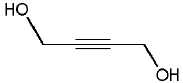 |
| 1,4-cyclohexanediol | 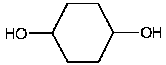 |
| 2,5-bis(hydroxymethyl)tetrahydrofurane | 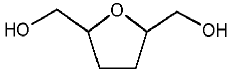 |
| 1,4-bis(hydroxyméthyl)cyclohexane | 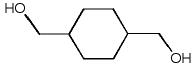 |
| Isosorbide | 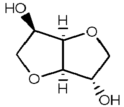 |
| 2,5-bis(hydroxymethyl)furane | 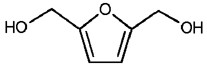 |
| 1,4-bis(hydroxymethyl)benzène | 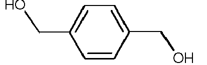 |
| Catéchol | 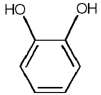 |
| Résorcinol | 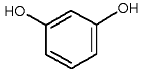 |
| Hydroquinone | 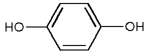 |
| 4,4'-dihydroxybiphenyle | 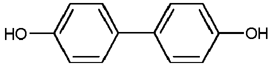 |
| 2,6-dihydroxynaphthalene | 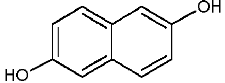 |

Dans un autre mode de réalisation avantageux de la demande, les dérivés polyesters d'acides gras de polyglycosides sont des biglycosides et répondent à la formule (Ia)

A-O-X-O-Z (Ia)

dans laquelle
- A et Z représentent chacun, indépendamment l'un de l'autre, un glucide réducteur choisi dans le groupe des monosaccharides comprenant le glucose, le fructose, le galactose et le mannose ou dans le groupe des disaccharides comprenant le lactose et le maltose,
   ∘ lesdits monosaccharides et lesdits disaccharides étant liés à-O-X-O- par le carbone anomérique portant initialement le OH hémiacétalique,
   ∘ l'un au moins des autres OH dudit monosaccharide ou dudit disaccharide étant estérifié par un dérivé lipidique porteur d'au moins une double liaison éventuellement issu d'une huile végétale ou animale ou d'un mélange d'huiles végétales ou animales,
   ∘ la double liaison ou l'une au moins des doubles liaisons dudit dérivé lipidique étant fonctionnalisée par un groupe choisi parmi les groupes époxy, amines, alcools et acides et
- X est la structure chimique porteuse des fonctions hydroxy dans un composé choisi dans le groupe comprenant les polyols aliphatiques, cycloaliphatiques, aromatiques.

Cette formule (Ia) est dérivée de la formule (I) en considérant m=2 donc n=0.

Ainsi lorsque X est la structure chimique porteuse des fonctions hydroxy
provenant respectivement du glycérol, du xylitol, de l'érythritol, du propanediol ou de l'hydroquinone, alors X représente respectivement : 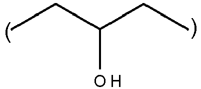 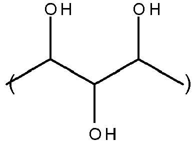 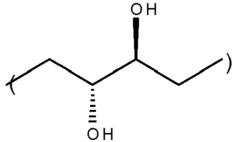 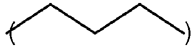 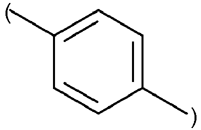 où (- et -) représentent le point d'attache de O-A et de O-Z et où les autres fonctions OH peuvent également être estérifiées par des mono- ou des disaccharides tels que ceux mentionnés précédemment.

Les dérivés lipidiques sont soit disponibles dans le commerce, soit préparés par des techniques connues de l'homme du métier. Les chaînes comprennent entre 4 et 36 atomes de carbone (chaîne en C₄-C₃₆), avantageusement entre 12 et 20 atomes de carbone (chaîne en C₁₂-C₂₀). Ils peuvent être issus d'huiles végétales parmi lesquelles on peut citer, les huiles végétales de lin, de tournesol, de colza, de chanvre, de sauge, de soja, d'olive, de pépins de raisins, de bois de Tung, de margose, de coton, de maïs, de noisette, de noix, de noix de coco, d'avocat, de palme, de ricin, de carthame, de germe de blé, de courge de Syrie, de noix de cajou et d'arachide ou d'huiles animales, comme par exemple le saindoux, le suif de boeuf, la graisse de canard et les huiles de poisson (saumon, sardine, anchois, maquereau, thon, hareng...). Conformément à l'invention, lorsqu'on utilise ces huiles, on peut utiliser les huiles seules ou des mélanges d'huiles indépendamment de leur origine, végétale ou animale.

Dans un mode de réalisation avantageux de l'invention, dans les dérivés polyesters d'acides gras de polyglycosides selon l'invention, le dérivé lipidique est choisi parmi les acides gras mono- ou polyinsaturés et les esters d'acides gras mono- ou polyinsaturés. On peut citer à titre d'exemple les acides gras et leurs dérivés comprenant entre 1 et 6 insaturations, avantageusement entre 2 et 3 insaturations. Selon l'invention, les acides gras sont choisis parmi l'acide arachidonique, l'acide docosahexaénoique, l'acide éicosapentaénoique, l'acide érucique, l'acide linoléique, l'acide linolénique, l'acide éléostéarique, l'acide nervonique, l'acide oléique, l'acide palmitoléique, l'acide ricinoléique, l'acide vernolique. Les dérivés d'acides gras sont notamment choisis parmi les esters d'acides gras, tels que par exemple l'oléate de méthyle ou le linoéate de méthyle, les amides gras obtenus par amidification des acides gras, et les thioesters gras issus de la thioestérification des acides gras, notamment des acides gras selon l'invention.

Dans un mode de réalisation avantageux de l'invention, dans les dérivés polyesters d'acides gras de polyglycosides, A et Z, identiques ou différents, représentent chacun une unité glucosique de formule (II) 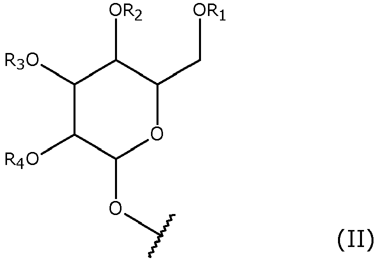 dans laquelle
R₁, R₂, R₃ et R₄, identiques ou différents représentent chacun :
   - soit un atome d'hydrogène,
   - soit un groupe -C(O)(C₄-C₃₆)alkyle, avantageusement un groupe -C(O)(C₁₂-C₂₀)alkyle, issu d'un acide gras saturé provenant d'une huile végétale ou animale ou d'un mélange d'huiles végétales ou animales,
   - soit un groupe -C(O)(C₄-C₃₆)alcényle, avantageusement un groupe -C(O)(C₁₂-C₂₀)alcényle, issu d'un acide gras insaturé provenant d'une huile végétale ou animale ou d'un mélange d'huiles, végétales ou animales, ledit groupe pouvant porter après fonctionnalisation chimique d'au moins une de ses doubles liaisons, un groupe fonctionnel choisi parmi les fonctions époxy, amines, alcools, et acides, et si plusieurs doubles liaisons sont fonctionnalisées, alors la fonctionnalité est identique pour toutes les doubles liaisons dudit groupe -C(O)(C₄-C₃₆)alcényle,
et à condition que l'un au moins de R₁, R₂, R₃ et R₄ soit un groupe -C(O)(C₄-C₃₆)alcényle fonctionnalisé tel que défini précédemment.

L'invention permet l'emploi parallèle de plusieurs acides ou esters d'acides gras. D'ailleurs, un dérivé biglucoside X selon l'invention pourrait être représenté par la formule générale (Ib) suivante : 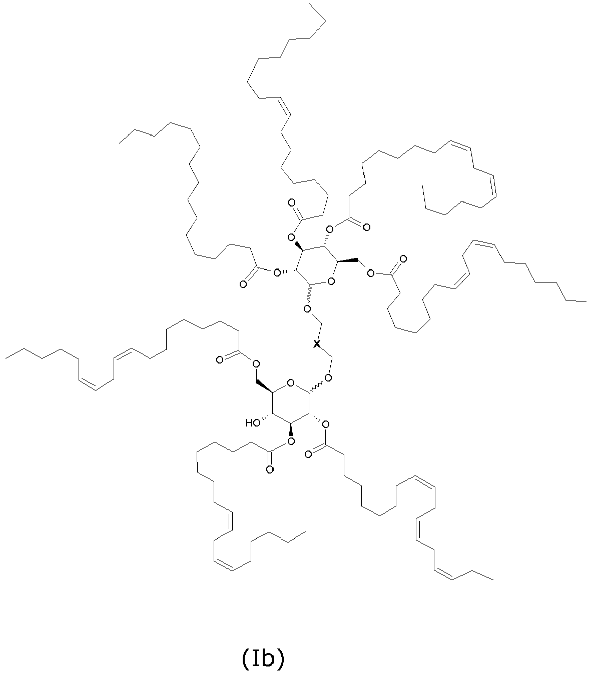 dans laquelle X est tel que défini précédemment. Cette formule présentée à titre d'exemple, correspond à une formule (Ia) dans laquelle A est un glucose porteur de 4 chaînes grasses dérivées respectivement du stéarate de méthyle (aucune insaturation), de l'oléate de méthyle (une insaturation), ou encore du linoéate de méthyle (deux insaturations) alors que Z est un glucose porteur de 3 chaînes grasses dérivées respectivement du linoéate de méthyle (deux insaturations) ou du linolénate de méthyle (trois insaturations).

Dans un mode de réalisation particulièrement avantageux de l'invention, X est un résidu de 1,3-propanediol ou un résidu d'hydroquinone.

Conformément à l'invention, les dérivés peuvent être préparés par toute technique connue de l'homme du métier, décrite dans la littérature à partir de produits disponibles dans le commerce ou décrits dans la littérature.

Ainsi ils peuvent être obtenus par exemple par un procédé comprenant 3 étapes :
- une étape de « glycosidation » du glycosyle donneur ou une étape de glycosylation de l'accepteur (le polyol) permettant d'obtenir le polyglycoside nu c'est à dire sans greffage des chaînes grasses,
- une étape de greffage des chaînes grasses, par exemple par interestérification dans le cas d'esters d'acide gras réagissant sur des unités glycosyle peracétylées et
- une étape de fonctionnalisation des doubles liaisons, par exemple par époxydation par la voie peracide reposant sur l'emploi combiné d'acide acétique, de peroxyde d'hydrogène en présence d'une résine échangeuse d'ions (type Amberlite 120H).

L'étape de synthèse des polyglycosides peut être réalisée par exemple par une des techniques suivantes :
- glycosylation d'aglycones de nature polyol avec un glycosyle donneur peracétylé catalysée par un acide de Lewis selon la méthodologie de Smits, E., et al. (J. Chem. Soc., Perkin Trans. 1, 1996 p. 2873-2877) ou celle décrite dans la demande internationale WO2004/007516, ou
- glycosidation impliquant des glycosyles donneurs comme par exemple des bromures, chlorures, thioimidates ou des thiocyanates et des catalyseurs comme par exemple SnCl₄, TMSOTf, FeCl₃ ou TrClO₄,
- pré-activation d'un aglycone,
- « chimie click » telle que décrite par Neto, V. et al., Tetrahedron, 2010, p. 4633-4646 et Marra, A., et al. J. Org. Chem., 2008., p. 2458-2461,
- couplage de Sonogashira-Heck-Cassar (palladium) selon la technique de Dondoni, A., *et al.* 2002, p. 1850-1854.
- métathèse catalysée par le Ruthénium selon la technique de Neto V. *et al.* (déjà cité) ou celle de Roy, R. et al. Chem. Commun. (Cambridge), 2000, p. 519-529,
- couplage d'Ullman (palladium) selon la technique de Bergeron-Brlek, M., et al. J. Org. Chem., 2012, p. 2971-2977,
- couplage d'Heck (palladium) selon la technique de Giguere, D., et al. Bioorg. Med. Chem., 2008, p. 7811-7823,
- couplage de Glaser selon la technique de Siemsen, P., et al. Angew. Chem., Int. Ed., 2000, p. 2632-2657.

La seconde étape peut être réalisée par exemple :
- par l'interestérification de bisglucosidesX peracétylés avec des dérivés lipidiques, par exemple avec des esters méthyliques d'acides gras (EMAG) comme l'oléate de méthyle, en présence de sodium ou d'un sel de sodium comme le méthanolate de sodium. Ces techniques sont décrites notamment par Akoh, C.C. et al. (J. Food Sci., vol. 55, 1990, p. 236-243 ; J. Food Sci., vol. 52, 1987, p. 1570-1576 ; J. Am. Oil Chem. Soc., vol. 66, 1989, p. 1581-1587), Mieth, G., et al. (Nahrung, vol. 27, 1983, p. 747-751) et dans les demandes DD156263 et US4973489 ou
- par transestérification entre des glucides non protégés avec des EMAG selon le procédé décrit dans la demande internationale WO9938875.

La troisième étape, est la fonctionnalisation des insaturations portées par les chaines grasses et qui peut être réalisées par diverses approches illustrées dans la figure ci-dessous et qui font appel à des techniques connues de l'homme du métier.

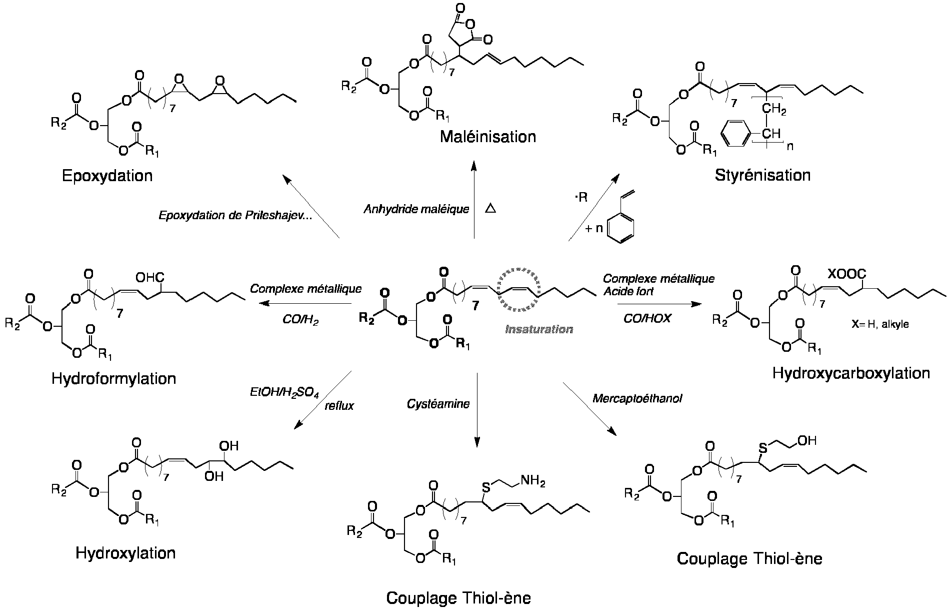

Lorsque la fonctionnalisation des doubles liaisons consiste en une époxydation, elle peut être réalisée par exemple par la technique décrite par Pan, X., et al., Green Chem., 2011, p. 965-975 et dans la demande internationale WO2011/097484.

Lorsque c'est un couplage thiol-ène (TEC : Thiol-Ene Coupling), la fonctionnalisation peut être réalisée par addition radicalaire sur les doubles liaisons C=C de mercaptoéthanol selon la technique de Desroches, M., et al. Polymer Reviews, 2012. 52(1): p. 38-79 ou de chlorhydrate de cystéamine (CAHC) selon la technique de Stemmelen, M., et al. Journal of Polymer Science Part a-Polymer Chemistry, 2011. 49(11): p. 2434-2444. Cette réaction peut être réalisée par voie thermique ou par voie photochimique sous UV.

La troisième étape peut également consister en :
- une styrénisation qui consiste à créer dans une première étape des radicaux sur les carbones allyliques grâce à la décomposition thermique d'un amorceur radicalaire comme le peroxyde de benzoyle. Les sites ainsi activés permettent par homopolymérisation du styrène d'implanter des greffons de polystyrène sur les chaînes d'acide gras selon la technique de Guner, F.S., et al. Progress in Polymer Science, 2006. 31(7): p. 633-670,
- une hydroformylation également appelée « oxo-procédé » qui permet d'introduire par l'intermédiaire des insaturations une fonction aldéhyde sur les chaines lipidiques en utilisant un complexe métallique, généralement, à base de cobalt selon la technique de Kandanarachchi, P., et al. Journal of Molecular Catalysis a-Chemical, 2002. 184(1-2): p. 65-71,
- une hydroxycarboxylation ou une réaction de Koch qui permettent d'introduire des fonctions acide carboxylique sur les doubles liaisons des segments lipidiques, selon la technique de Corma, A., et al., Chemical Reviews, 2007. 107(6): p. 2411-2502,
- une maléinisation qui permet de greffer des unités d'anhydride maléique sur les chaines grasses selon la technique de Stefanoiu, F., et al. European Journal of Lipid Science and Technology, 2008. 110(5): p. 441-447.

Les dérivés selon l'invention peuvent être utilisés pour réaliser des formulations liquides ou solides qui sont nouvelles et font parties de l'invention. On entend par « formulation » un mélange d'au moins deux composés réactifs. Par exemple, selon cette terminologie, une formulation époxy désignera le mélange réactionnel d'au moins une molécule fonctionnalisée époxy (=prépolymère) avec au moins un durcisseur (polyamine, polyacide, anhydride cyclique...)

Aussi l'invention a également pour objet des formulations liquides ou solides comprenant au moins un dérivé selon l'invention et éventuellement un agent de réticulation.

Lorsque la troisième étape est une époxydation, alors on obtient un prépolymère époxy apte à entrer dans la composition de résines thermodurcissables et par la même de permettre la production de matériaux par réaction avec divers agents de réticulation biosourcés ou non tels que les anhydrides de diacides, les composés porteurs d'amines primaires ou secondaires comme les diamines, les polyamines et leurs mélanges, avantageusement les diamines, les diacides et les polyacides, les alcools, y compris les phénols, et les polymercaptans.

On peut citer à titre d'exemple d'anhydrides de diacides : l'anhydride succinique, l'anhydride maléique, l'anhydride dodécénylsuccinique, l'anhydride phtalique, l'anhydride hexahydrophtalique, l'anhydride méthylhexahydrophtalique, l'anhydride méthyl-tétrahydrophtalique, l'anhydride méthyl-endo-méthylènetétrahydrophtalique, l'anhydride de l'acide citrique, l'anhydride de l'acide oxalique, l'anhydride itaconique et l'anhydride aconitique.

On peut citer à titre d'exemple d'amines, d'origine biosourcées ou non, les diamines aliphatiques comme par exemple l'éthylènediamine, l'hexaméthylènediamine, la bis(3-aminopropyl)amine, la 1,10-décanediamine, la 1,4-butanediamine, la 1,5-pentanediamine, la 1,12-dodécanediamine ou la 1,18-octadécanediamine, les diamines cycloaliphatiques comme l'isophorone diamine (IPDA), les diamines aromatiques comme la phénylènediamine sous ses formes ortho, méta, para, la xylylènediamine sous ses formes ortho, méta, para, le 2,5-diaminotoluène, le 4,4'-diaminobiphényle, le 4,4'-diaminodiphénylméthane ou la lysine, les polyamines porteuses d'au moins 5 groupements N-H, notamment la diéthylènetriamine, la triéthylènetétramine, la tétraéthylènepentamine, la poly(oxypropylène) triamine et les polyétheramines, les polyoxyalkylèneamines ou les polypeptides naturels.

On peut citer à titre d'exemple de diacides, l'acide heptanedioïque, l'acide phtalique, l'acide isophtalique, l'acide fumarique, l'acide maléique, l'acide téréphtalique, l'acide succinique, l'acide itaconique, l'acide aconitique, l'acide hexahydrophtalique, l'acide méthyl hexahydrophtalique, l'acide tétrahydrophtalique, l'acide méthyl tétrahydrophtalique et l'acide pyroméllitique.

On peut citer à titre d'exemple de polymercaptans ou de polythiols, le 1,2,5-trimercapto-4-thiapentane, 3,3-dimercaptomethyl-1,5-dimercapto-2,4-dithiapentane, le 3-mercapto-méthyl-1,5-dimercapto-2,4-dithiapentane, 3-mercaptométhylthio-1,7-dimercapto-2,6-dithiaheptane, 1,2,7-trimercapto-4,6-dithiaheptane, 3,6-dimercaptométhyl-1,9-dimercapto-2,5,8-trithianonane, 1,2,9-trimer-capto-4,6,8-trithianonane, le 3,7-dimercaptomethyl-1,9-dimercapto-2,5,8-trithianonane, le 4,6-dimercaptomethyl-1,9-dimercapto-2,5,8-trithianonane, le 3-mercaptomethyl-1,6-dimercapto-2,5-dithiahexane, le 3-mercap-tométhylthio-1,5-dimercapto-2-thiapentane, le 1,1,2,2-tetrakis (mercaptométhylthio)éthane, 1,1,3,3-tetrakis(mercaptomethylthio)propane, le 1,4,8,11-tetramercapto-2,6,10-trithiaundecane, 1,4,9,12-tetramercapto-2,6,7,11-tetrathiadodecane, le 2,3-dithia-1,4-butanedithiol, et le 2,3,5,6-tetrathia-1,7-heptanedithiol, 2,3,5,6,8,9-hexathia-1,10-decanedithiol....

L'invention a également pour objet des résines époxydes biosourcées comprenant le produit de réaction d'un ou de plusieurs dérivés selon l'invention avec au moins un agent de réticulation et éventuellement en présence d'au moins un co-réactif choisi parmi les dérivés glycidyl éthers de polyols biosourcés tels ceux décrits dans la demande internationale WO2013/124574.

Une des voies privilégiées de fonctionnalisation est celle basée sur la chimie thiolène (couplage thiol-ène sur la figure ci-dessus) qui consiste à pouvoir faire réagir sur les insaturations un greffon R-SH où R est un segment moléculaire permettant l'implantation d'une nouvelle terminaison chimique. Par exemple, l'utilisation de cystéamine permet de greffer sur les chaines lipidiques de courts segments moléculaires terminés par des groupements amine et par la même, de décliner la molécule plateforme selon l'invention en polyamine. Cette polyamine peut notamment être valorisée en tant qu'agent de réticulation de résines époxy. Ainsi, il est possible de produire une formulation reposant totalement sur les dérivés selon l'invention puisque la structure polyester lipidique peut être fonctionnalisée à façon. Un dérivé peut être utilisé pour produire le prépolymère époxy quand un autre peut être modifié par chimie thiolène pour synthétiser l'agent de réticulation de type polyamine. Après le mélange des deux molécules, préférentiellement en quantité stoechiométrique (isoproportion de groupements époxy et groupements amine), il est possible par réaction de réticulation de produire un matériau dont les performances ultimes dépendent :
- de la fonctionnalité de l'espaceur central (diol ou polyol),
- de sa nature (aliphatique, aromatique ou cycloaliphatique),
- de la nature même des unités glycosidiques réductrices choisies,
- de la quantité de chaines lipidiques insaturées initialement greffées sur les unités glycosidiques,
- du nombre d'insaturations portées par chaque chaine grasse (compris entre 1 et 6)
- de la fonctionnalisation chimique (amine, acide carboxylique, alcool...) de ces mêmes insaturations pour la production de l'agent de réticulation
- du degré d'époxydation (cas de production du prépolymère)

L'invention permet de produire des solutions alternatives à de nombreuses formulations pétrochimiques que ce soit au travers du développement d'architectures prépolymères tels que les polyépoxy ou des différentes classes de durcisseurs (polyamines, polyanhydrides, polyacides...). D'autres classes de polymères sont accessibles à l'image des polyuréthanes ou des polyamides par l'avènement des structures polyols. Le marché potentiel est celui couvert par les polymères pétrosourcés notamment sur les applications techniques où de nombreux produits sont ou vont être frappés d'obsolescence car toxiques (matrices de composites, adhésifs, peintures, vernis, isolants électriques...). Sur le seul volet des prépolymères époxy, l'axe fort de l'invention est la production de structures rigides sans épichlorohydrine et sans Bisphénol A.

La présente invention a donc pour objet l'utilisation de ces dérivés comme prépolymères pour la fabrication de polymères choisis dans le groupe comprenant en particulier les polyesters, les polyamides, les polyuréthanes et les résines époxy.

Ces polymères peuvent être préparés à partir des dérivés selon la présente invention par toute technique connue de l'homme du métier, notamment par modification chimique ou physique.

Les modes de mise en oeuvre sont relativement nombreux. Si on ne retient que ceux classiquement employés avec les thermodurs, on peut citer les procédés type SMC (Moulage de pré-imprégnés en feuilles ou Sheet Moulding Compound), BMC (Moulage de composés pré-imprégnés en masse ou Bulk Moulding Composite), préimprégné, RTM (moulage par injection de résine liquide ou Resin Transfert Molding), pultrusion, infusion, le thermoformage ou encore l'enroulement filamentaire. Ces procédés nécessitent l'emploi d'une résine de viscosité bien définie et qui peut être modulée selon notre invention par la nature et la fonctionnalité du coeur central, la nature des unités glycosidiques réductrices retenues, la quantité des chaines lipidiques greffées sur chaque unité glucosidique ainsi que la densité et la nature de la fonctionnalisation chimique de leur insaturation. Par exemple, à greffage et fonctionnalité identiques, les molécules dotées d'un coeur aromatique afficheront une viscosité nettement plus élevée que celle observée avec les molécules dont la structure centrale est aliphatique. La densité de la fonctionnalisation est un autre paramètre efficace pour ajuster la viscosité initiale de la résine (voir exemple 1 dans le cas de systèmes époxy). Il est ainsi possible de préparer des molécules qui vont produire des solutions fluides parfaitement adaptées aux processus d'imprégnation directe (infusion, RTM, enroulement filamentaire...) ou inversement des résines dotées d'une forte viscosité nécessaires à leur emploi par les techniques des SMC, BMC voire préimprégnés. Les exemples 5 et 6 illustrent notamment ce concept au travers de l'application de notre invention au développement de prépolymères époxy.

Aussi l'invention a pour objet des matériaux obtenus par modification chimique ou physique d'une formulation selon l'invention.

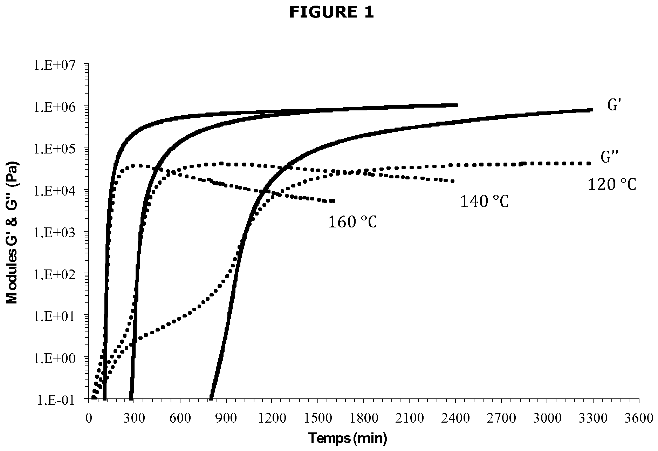

Les exemples 1 à 6 et les figures 1 à 3 qui suivent illustrent l'invention sans toutefois la limiter.

La figure 1 illustre la cinétique de réticulation du mélange BGH8E-IPDA préparé selon l'exemple 4 pour différentes températures de réticulation, 120 °C, 140 °C et 160 °C. Les courbes représentent l'évolution cinétique des composantes viscoélastiques dudit mélange réactionnel. La composante G' (courbes en trait continu) est appelée «module de conservation» ; elle traduit l'énergie stockée puis restituée par le matériau et illustre sa rigidité mécanique. La composante G" (courbes en pointillé) désigne le «module de perte» caractéristique de l'énergie mécanique dissipée du fait des mouvements moléculaires se produisant au sein de la matière.

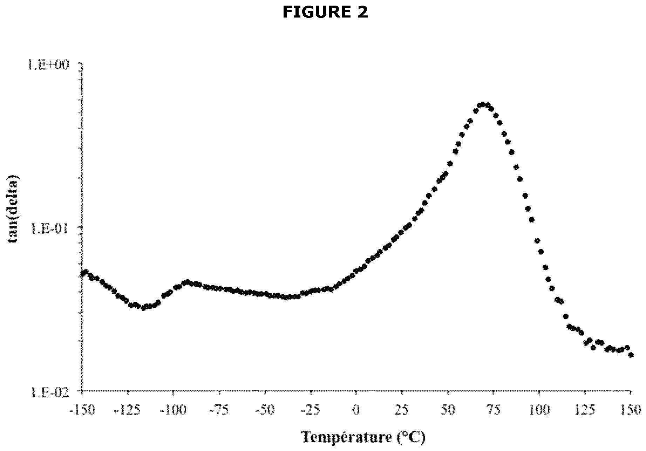

La figure 2 illustre le profil thermomécanique du matériau BGH8E-MHPPA obtenu selon l'exemple 5 après réticulation durant 3 heures à 140 °C. La courbe, appelée tan(delta), est le rapport G"/G' mesuré à différentes températures.

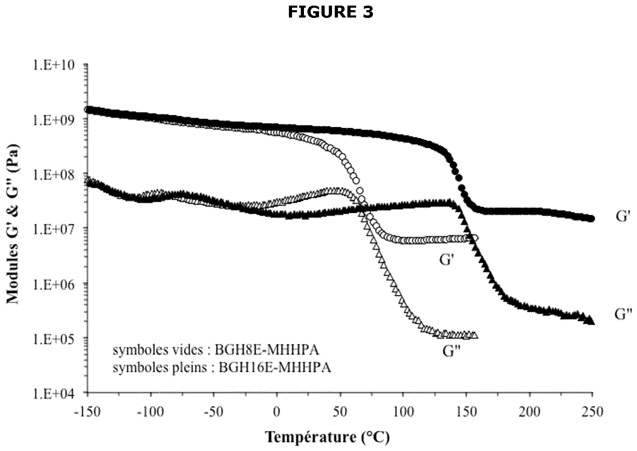

La figure 3 est une comparaison des réponses thermomécaniques des matériaux BGH8E-MHHPA et BGH16E-MHHPA obtenus respectivement selon les exemples 5 et 6. Les symboles vides désignent la réponse du BGH8E-MHHPA et les symboles pleins désignent la réponse du BGH16E-MHHPA. L'évolution de la composante G' est représentée par les symboles circulaires (○,●) alors que celle de la composante G" est représentée par les symboles triangulaires (△,▲).

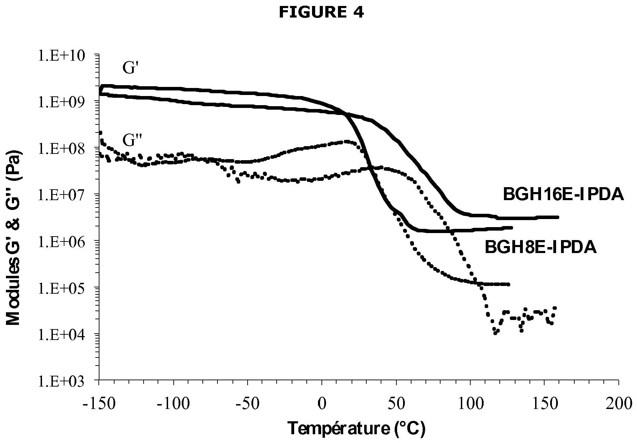

La figure 4 rassemble les profils thermomécaniques des matériaux obtenus par réticulation respective des formulations réactives BGH8E-IPDA et BGH16E-IPDA. Les courbes en trait continu sont caractéristiques du «module de conservation» G' de chaque matrice alors que les courbes en pointillé représentent l'évolution du «module de perte» G" de ces mêmes matières. La température de transition vitreuse peut être évaluée en relevant la température de relaxation principale c'est à dire au maximum de la courbe G". Cette série permet d'analyser l'effet induit par la nature de la chaine lipidique et indirectement par le nombre de fonctions époxy « x » par unité moléculaire de BGHxE sur les performances ultimes des matrices ayant utilisé l'IPDA comme durcisseur.

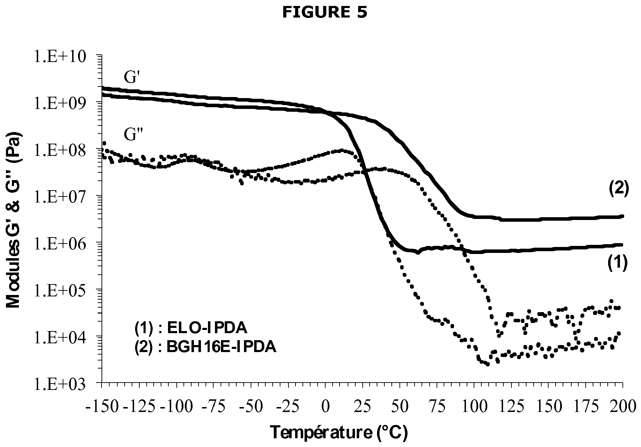

La figure 5 permet la comparaison directe du profil thermomécanique de la matrice réticulée BGH16E-IPDA avec celui caractéristique du matériau obtenu à partir du mélange stoechiométrique d'huile de lin époxydée (ELO) durcie avec le même agent de réticulation (IPDA). Les courbes en trait continu sont caractéristiques du «module de conservation» G' de chaque matrice alors que les courbes en pointillé représentent l'évolution du «module de perte» G". La température de transition vitreuse est à nouveau évaluée au maximum de la courbe G". Au delà du nombre de fonctions époxy par molécule (16 pour le BGH16E et 6 pour l'ELO), cette figure illustre surtout l'influence du coeur moléculaire du prépolymère époxy (BGH dans le cas du BGH16E et glycérol dans le cas de l'ELO) sur les propriétés thermomécaniques de chaque matrice après réticulation.

### EXEMPLE 1 : Synthèse générale d'un polyesters d'acide gras de biglycosides

### 1.1. Préparation du biglucosideX peracétylé

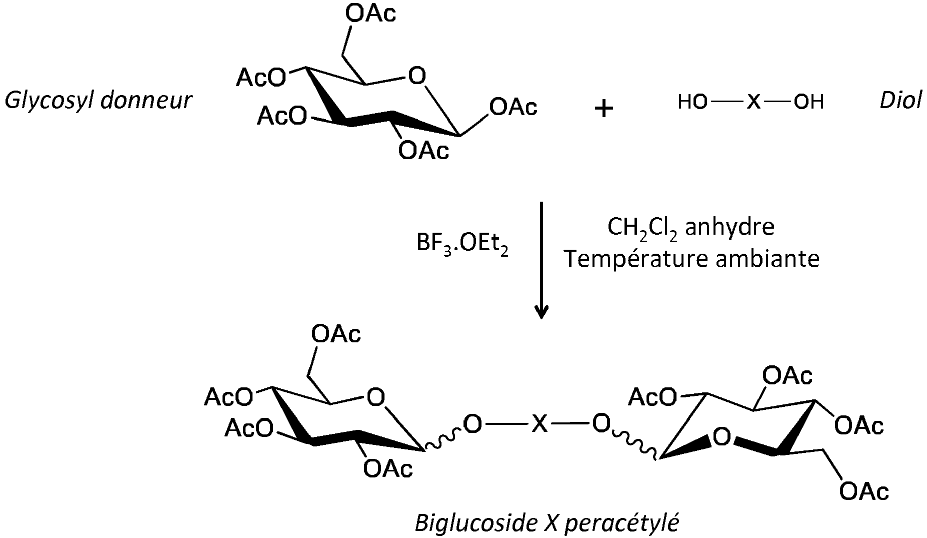

Conformément au schéma ci-dessus, le β-D-Glucose pentaacétate (β-D-Glc(OAc)₅) est utilisé comme glycosyl donneur. Il existe commercialement ou peut être aisément obtenu par action de l'anhydride acétique sur le glucose (selon la technique de la peracétylation connue de l'homme du métier). Le diol est représenté par la formule HO-X-OH. Le glycosyl donneur et le diol sont solubilisés dans le dichlorométhane (CH₂Cl₂) anhydre. Ensuite l'éthérate de trifluorure de bore (boron trifluoride etherate, BF₃.OEt₂) est additionné goutte à goutte au mélange réactionnel dans un rapport molaire glycosyl donneur / HO-X-OH / BF₃.OEt₂ de 1 : 0,5 : 1. Le milieu réactionnel est agité à température ambiante pendant 24 h sous atmosphère inerte (azote). La phase organique est purifiée par extraction liquide-liquide avec une solution aqueuse saturée d'hydrocarbonate de sodium (NaHCO₃) puis de chlorure de sodium (NaCl). La phase organique est séchée avec du sulfate de magnésium (MgSO₄) puis concentrée par évaporation du CH₂Cl₂ sous pression réduite. Les biglucosides X (également désignés sous le terme de bisglucosides et codés par BGX) sont isolés par chromatographie sur colonne ou par recristallisation selon la nature du diol.
Les rendements des bisglucosides BGX et des sous-produits éventuels dépendent de la nucléophilie du diol.

### 1.2. Interestérification (échange ester-ester)

La réaction mise en jeu lors de la synthèse de bisglucosides Polyesters d'acides gras par interestérification est donnée dans le schéma ci-dessous où R représente une chaîne lipidique et R₁ un bisglucoside

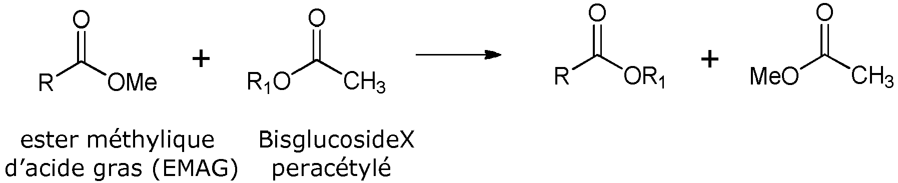

Le bisglucoside X peracétylé obtenu à l'étape 1.1, l'ester méthylique d'acide gras (EMAG) et 2% de Na exprimé en masse total du mélange sont mélangés et l'interestérification est réalisée à 110-120 °C pendant 2-6 h sous une pression réduite comprise entre 0 et 667 Pa (0-5 mm Hg) afin de piéger l'acétate de méthyle formé. Le rapport molaire groupement Acétate : EMAG est de 1 : 1.

En fin de réaction, le mélange réactionnel est dilué à chaud avec de l'hexane puis neutralisé avec de l'acide acétique. La phase organique est purifiée par extraction liquide-liquide avec du méthanol. Les Bisglucosides Polyester avec des DS élevés de 5-8 sont isolés après évaporation de l'hexane sous pression réduite. Le degré de substitution moyen du mélange est déterminé par RMN ¹H et MALDI.

### 1.3. Fonctionnalisation des doubles liaisons

### 1.3.1. Epoxydation

Les bisglucosides polyesters d'acides gras (BGP) possédant « *n* » moles de doubles liaisons obtenus selon l'étape 1.2, l'acide acétique et la résine Amberlite 120H (20% en masse de la masse de BGP) sont introduits dans un réacteur avec agitation mécanique. Le ratio molaire acide acétique / H₂O₂ / double liaisons est de 0,5 : 2 : 1. Le mélange réactionnel est mélangé sous atmosphère inerte et chauffé à environ 60-70 °C afin de réduire la viscosité du BGP et ainsi obtenir un mélange homogène. Le peroxyde d'hydrogène (H₂O₂; 50% en masse en solution aqueuse) est additionné goutte à goutte. Après addition, le mélange réactionnel est mélangé pendant 30 min à 4 h selon le degré d'époxydation recherché. En fin de réaction, le mélange réactionnel est dilué avec de l'éther diéthylique puis la résine est filtrée. Le mélange est purifié par extraction liquide-liquide avec H₂O, puis avec une solution aqueuse saturée de NaHCO₃ puis de NaCl. La phase organique est séchée avec MgSO₄ puis évaporée sous pression réduite pour récupérer les bisglucosides polyesters d'acides gras époxydé (BGPE). Les rendements isolés sont tous supérieurs à 95%.

### 1.3.2. Fonctionnalisation par chimie thiol-ène

### - Voie thermique

Cette méthode nécessite l'emploi d'un amorceur (2,2'-azobisisobutyronitrile ou AIBN) à hauteur de 0,02 éq pour 3 éq. d'hydrochlorure de cystéamine (3 éq). Cette dernière est dosée de telle sorte que le ratio molaire [CAHC]/[C=C] soit égal à 3. Le BisGlucoside Polyesters d'acides gras insaturés préparé selon le protocole de l'étape 1.3.2, l'AIBN et le CAHC sont mis en solution dans un mélange 1,4-dioxane-éthanol (70-30). Le mélange réactionnel est chauffé à 80°C, sous agitation, pendant 24 heures puis est filtré sur Büchner. Le filtrat est concentré sous vide puis solubilisé dans du chloroforme (100 mL). La solution est lavée avec une solution saturée en NaCl (5 x 350 mL). La phase organique est séchée avec MgSO4, puis concentrée sous vide. Le rendement est de l'ordre de 70%.

### - Voie photochimique

Dans un erlenmeyer, le BisGlucoside Polyesters d'acides gras insaturés préparé selon le protocole de l'étape 1.3.2, l'hydrochlorure de cystéamine (3 éq par doubles liaisons présentes sur les chaines lipidiques) et un photoamorceur (2,2-diméthoxy-2-phénylacetophenone - DMPA dosé à 0,1 éq par [C=C]) sont mis en solution dans un mélange 1,4-dioxane/éthanol (selon le ratio massique 70/30 m/m). Le mélange est légèrement chauffé à une température maximale de 40 °C puis est laissé sous agitation mécanique jusqu'à dissolution de l'hydrochlorure de cystéamine. Puis le mélange est versé dans un photoréacteur sous agitation et irradiation UV pendant une durée comprise entre 8 et 96 h (variable suivant le niveau de fonctionnalisation désiré). La température est maintenue à une valeur de 20 °C au moyen d'un système réfrigérant adapté. Pour un faible taux de conversion, l'éthanol peut être évaporé et la phase organique est lavée avec une solution saturée en Na₂CO₃. Elle est alors placée à -20 °C pendant 24 h pour faire recristalliser l'hydrochlorure de cystéamine. Ce dernier est alors éliminé par filtration sur Büchner (fritté de porosité 4). Les étapes de recristallisation-filtration sont répétées jusqu'à ce qu'il n'y ait plus d'hydrochlorure de cystéamine à recristalliser. La solution est alors lavée avec une solution saturée en Na₂CO₃ (3 x 150 mL). La phase organique est séchée avec MgSO₄, puis concentrée sous vide. Pour obtenir un taux de conversion élevé, il est nécessaire de remplacer les solvants par du chloroforme. La phase organique est lavée et isolée comme précédemment. Le taux de conversion est de l'ordre de 87 à 90 %

### EXEMPLE 2 : Synthèse du BGH8

Le BGH8 de formule : 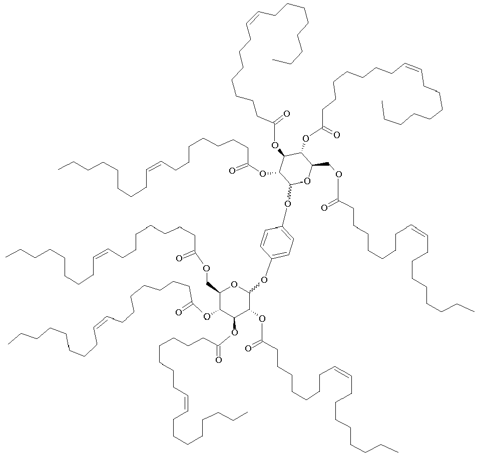 a été synthétisé selon le procédé général de l'exemple 1 (étapes 1.1 et 1.2) à partir de glucose, d'hydroquinone et d'oléate de méthyle.

### EXEMPLE 3 : Synthèse du BGH8E

Ce composé BGH8E de formule 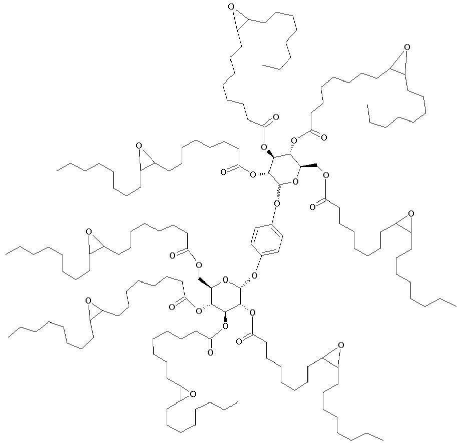 est préparé par époxydation du BGH8 de l'exemple 2 selon le protocole décrit à l'exemple 1.3.1.

### EXEMPLE 4 : Résine époxy préparée à partir de BGH8E et de durcisseur IPDA (BGH8E-IPDA)

### 4.1. Préparation de la résine

Dans cet exemple, la résine est formée par le mélange réactionnel du prépolymère époxy BGH8E avec une diamine cycloaliphatique, l'isophorone diamine (IPDA). Le dosage du prépolymère et du durcisseur est effectué afin de garantir une consommation totale des espèces réactives. Cette dernière nécessite que le nombre total de groupements amine N-H soit égal au nombre total de groupements époxy présents dans le milieu. Le BGH8E étant porteur de 8 fonctions époxy par molécule alors que la diamine IPDA comporte 4 fonctions NH par molécule, le dosage molaire associé est 1:2. Cela revient à dire qu'à 100 parts de BGH8E dérivé de biomasse sont associées 12,7 parts de diamine IPDA dérivée de la pétrochimie. La proportion de carbone fossile est donc faible.

Concernant la phase de mélange proprement dite, le prépolymère époxy BGH8E est préalablement chauffé à la température de 50 °C afin de présenter une faible viscosité. La diamine IPDA est liquide dès l'ambiante mais est chauffé à la même température pour permettre son incorporation et son mélange plus aisés avec le prépolymère.

La diamine est versée dans le prépolymère. Le brassage mécanique est mené durant 5 minutes alors que la température du mélange BGH8-IPDA est maintenue constante et égale à 50 °C.

Le mélange réactionnel est alors versé dans un moule adéquat avant la conduite de la réticulation à une température comprise dans le domaine réactionnel dont les limites de ce dernier sont préalablement déterminées par analyse calorimétrique.

### 4.2. Résultats du BGH8E-IPDA

L'étude de la cinétique de réticulation est menée au moyen d'analyses rhéologiques isothermes sur le mélange réactionnel. Les résultats obtenus à trois températures spécifiques sur des mélanges neufs sont présentés sur la figure 1.

Il est ainsi possible de définir avec soin les durées minimales de réticulation associées à chaque température de cuisson. Le matériau BGH8E-IPDA a ainsi été produit dans des conditions de temps et température optimisées.

Ainsi, après réticulation complète avec un durcisseur amine, BGH8E, qui compte 8 groupements époxy, permet d'obtenir un matériau dont les performances sont équivalentes à celles observées à durcisseur équivalent avec de la SEFOSE11 époxydée (SEFOSE11E) de formule 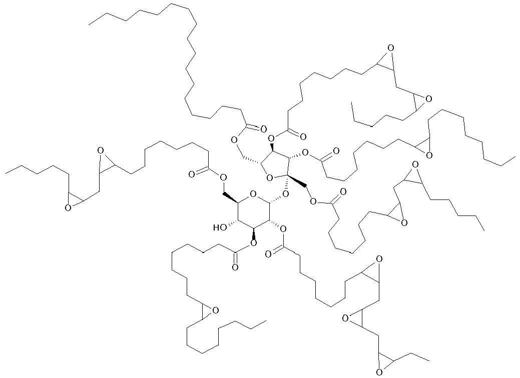 décrite dans la demande internationale WO 2011/097484 et qui comprend 11 groupes époxy. En effet, le matériau BGH8E-IPDA affiche une Tg de 18 °C contre 22 °C pour le matériau SEFOSE11E-IPDA.

### EXEMPLE 5 : Résine époxy préparée à partir de BGH8E et de durcisseur MHHPA (BGH8E- MHHPA)

### 5.1. Préparation de la résine

Dans ce nouvel exemple, la résine est préparée à partir du mélange entre le prépolymère époxy BGH8E et un agent de réticulation de type anhydride (le méthylhexahyphtalique anhydride désigné sous l'acronyme MHHPA). Le dosage du prépolymère et du durcisseur est effectué selon un ratio du nombre groupements anhydride sur le nombre de groupements époxy égal à 0,8 afin d'éviter la présence résiduelle de fonctions acide carboxylique néfastes aux propriétés physicochimiques du matériau. Sur le plan molaire, cela revient à doser 6,4 moles de fonctions anhydride pour une mole de BGH8E. En termes de proportions massiques, à 100 parts de BGH8E sont associées 40,1 parts d'anhydride MHHPA. 0,4 part de catalyseur de type 2-méthyl-imidazole est ajoutée au mélange réactionnel.

En ce qui concerne la phase de mélange proprement dite, le prépolymère époxy BGH8E est chauffé préalablement à la température de 50 °C afin de présenter une faible viscosité. Bien que liquide dès l'ambiante, l'anhydride liquide est chauffé à la même température afin de permettre sa meilleure incorporation et faciliter son mélange avec le prépolymère.

L'anhydride est ainsi ajouté au prépolymère puis le brassage mécanique est mené durant 5 minutes à une température constante et égale à 50 °C. Le catalyseur est enfin ajouté et le mélange est brassé mécaniquement durant 1 minute supplémentaire.

Après détermination des conditions optimales de réticulation selon le même protocole que celui présenté dans l'exemple 4, le mélange réactionnel est placé dans une enceinte thermique afin de polymériser.

### 5.2. Résultats du BGH8E-MHPPA

Le matériau à base de BGH8E affiche des caractéristiques thermomécaniques bien supérieures à celles enregistrées avec la formulation basée sur le mélange de la SEFOSE11E et du même durcisseur.

La figure 2 démontre qu'après réticulation durant 3 heures à 140 °C, le pic de relaxation mécanique du matériau est de l'ordre de 70 °C alors que la SEFOSE11E comportant 11 fonctions époxy affiche après réticulation avec le MHHPA une température de 48,4 °C comme reportée dans Pan et al., Biomacromolecules, 2011. 12(6): p. 2416-2428.

Le coeur central aromatique conférant de la rigidité au squelette chimique contribue à la température de transition vitreuse plus élevée. Ce résultat souligne l'importance de la modulation de la structure centrale puisque notre molécule produit de meilleurs résultats en dépit d'un nombre plus réduit de fonctions époxy.

Inversement, l'emploi d'un diol central aliphatique permet de produire un matériau très souple.

### EXEMPLE 6 : Résine époxy préparée à partir de BGH16E et de durcisseur MHHPA (BGH16E- MHHPA)

### 6.1. Préparation de la résine

### 6.1.1. Préparation du BGH16E

Comme précisé précédemment, notre invention autorise l'utilisation d'une large gamme d'esters d'acide gras. Ainsi, en utilisant du linoéate de méthyle en lieu et place de l'oléate de méthyle, il est possible de produire un bisglucoside époxydé comportant deux fonctions époxy par chaîne grasse soit un nombre total de 16 fonctions époxy par molécule. On utilise le procédé général de l'exemple 1 (étapes 1.1 et 1.2) à partir de glucose, d'hydroquinone et de linoéate de méthyle.

### 6.1.2. Préparation de la résine

Elle est préparée selon le mode opératoire de l'exemple 5 à partir de BGH16E et de MHPPA. Le dosage du prépolymère et du durcisseur est effectué selon un ratio du nombre groupements anhydride sur le nombre de groupements époxy égal à 0,8 afin d'éviter la présence résiduelle de fonctions acide carboxylique néfastes aux propriétés physicochimiques du matériau. Sur le plan molaire, cela revient à doser 12,8 moles de fonctions anhydride pour une mole de BGH16E. En termes de proportions massiques, à 100 parts de BGH16E sont associées 72,3 parts d'anhydride MHHPA. 0,7 part de catalyseur de type 2-méthyl-imidazole est ajoutée au mélange réactionnel.

### 6.2. Résultats du BGH16E-MHPPA

Cette polyvalence de synthèse est intéressante car si les autres postes de modulation des propriétés sont maintenus inchangés (i.e. un coeur aromatique dérivé d'hydroquinone et d'unités glucosiques, un durcisseur MHHPA) le niveau de performances final est encore plus élevé qu'avec le matériau BGH8E-MHHPA comme illustré dans la figure 3. Le pic de relaxation mécanique associé à la transition vitreuse du matériau, relevé au maximum de la courbe du module de perte G", augmente ainsi de 49 °C pour le BGH8E-MHHPA à 137 °C pour le BGH16E-MHHPA.

### EXEMPLE 7 : Résine époxy préparée à partir de BGH16E et de durcisseur IPDA (BGH16E-IPDA)

### 7.1. Préparation de la résine

Le BGH16E est préparé selon le mode opératoire de l'exemple 6. Son mélange avec le durcisseur IPDA est mené selon le protocole déjà détaillé à l'exemple 4 avec le couple BGH8E-IPDA. Le dosage du prépolymère et du durcisseur est effectué afin de garantir une consommation totale des espèces réactives. Cette dernière nécessite que le nombre total de groupements amine N-H soit égal au nombre total de groupements époxy présents dans le milieu. Le BGH16E étant porteur de 16 fonctions époxy par molécule alors que la diamine IPDA comporte 4 fonctions NH par molécule, le dosage molaire associé est 1:4. Cela revient à dire qu'à 100 parts de BGH16E biosourcé sont associées 24,4 parts de diamine IPDA dérivée de la pétrochimie.

### 7.2. Résultats du BGH16E-IPDA

Ils sont donnés dans les figures 4 et 5.

La figure 4 permet d'évaluer les performances thermomécaniques du matériau réticulé à partir de la formulation réactive BGH16E-IPDA. Le pic de relaxation mécanique associé à la transition vitreuse du matériau, relevé au maximum de la courbe du module de perte G", fournit une bonne évaluation de la température de transition vitreuse de ce matériau. Elle est de l'ordre de 42 °C pour le BGH16E-IPDA contre 18 °C pour le BGH8E-IPDA décrit précédemment dans l'exemple 4. On voit donc l'effet apporté directement par des chaînes linoléiques (cas du BGH16) en lieu et place de chaînes oléiques (cas du BGH8). Un degré d'insaturation plus élevé (BGH16E par rapport à BGH8E) permet un taux d'époxydation supérieur et par la même une maille macromoléculaire plus compacte, gage de performances thermomécaniques supérieures. Le matériau BGH8E sera au contraire plus intéressant que le BGH16E si le substrat réticulé doit afficher une plus grande souplesse mécanique.

La figure 5 permet la comparaison directe de la matrice BGH16E-IPDA avec celle issue d'huile de lin époxydée (ELO) comportant 6 fonctions époxy telle que décrite dans la demande internationale WO2012136940. Le dosage de l'ELO avec l'IPDA est mené selon un ratio molaire 1 : 1,5 et sa réticulation est opérée de manière complète à 140 °C durant 24 h. La supériorité de performances du matériau BGH16E-IPDA (Tg = 42 °C) est indéniable par comparaison à celles du ELO-IPDA (Tg = 11 °C). Les propriétés supérieures du BGH16E-IPDA sont induites par le nombre de fonctions époxy plus élevé et surtout par la présence du coeur central bisglucosidique dont la rigidité moléculaire est plus élevée que celle du coeur glycérol de l'ELO.

## Revendications

1. Dérivés polyesters d'acides gras de polyglycosides formés à partir d'un polyol comprenant entre 2 et 10 fonctions hydroxy, les 2 fonctions hydroxy ou au moins 2 de ces fonctions hydroxy étant liées au carbone anomérique d'un glucide réducteur, identique ou différent pour chaque groupe hydroxy et choisi parmi les monosaccharides et les disaccharides, dans lesquels l'un au moins des autres hydroxy dudit monosaccharide ou dudit disaccharide est estérifié par un dérivé lipidique porteur d'au moins une double liaison éventuellement issu d'une huile végétale ou animale ou d'un mélange d'huiles végétales ou animales, la double liaison ou l'une au moins des doubles liaisons dudit dérivé lipidique étant fonctionnalisée par un groupe choisi parmi les groupes époxy, amines, alcools et acides.

2. Dérivés polyesters d'acides gras de polyglycosides selon la revendication 1 caractérisés en qu'ils répondent à la formule (I) 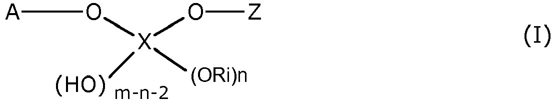 dans laquelle
- A et Z représentent chacun, indépendamment l'un de l'autre, un glucide réducteur choisi dans le groupe des monosaccharides comprenant le glucose, le fructose, le galactose et le mannose ou dans le groupe des disaccharides comprenant le lactose et le maltose,
∘ lesdits monosaccharides et lesdits disaccharides étant liés à -O-X-O- par leur carbone anomérique portant initialement le OH hémiacétalique,
∘ l'un au moins des autres OH dudit monosaccharide ou dudit disaccharide étant estérifié par un dérivé lipidique porteur d'au moins une double liaison éventuellement issu d'une huile végétale ou animale ou d'un mélange d'huiles végétales ou animales,
∘ la double liaison ou l'une au moins des doubles liaisons dudit dérivé lipidique étant fonctionnalisée par un groupe choisi parmi les groupes époxy, amines, alcools et acides,
- X représente une structure chimique porteuse des fonctions hydroxy dans un composé choisi dans le groupe comprenant les polyols aliphatiques (chaînes non cycliques), cycloaliphatiques et aromatiques,
- Ri représente soit un substituant, soit plusieurs substituants notés Rₐ à Rₕ, lesdits substituants Rₐ à Rₕ, identiques ou différents, étant des glucides réducteurs choisis dans le groupe des monosaccharides comprenant le glucose, le fructose, le galactose et le mannose ou dans le groupe des disaccharides comprenant le lactose et le maltose,
∘ lesdits monosaccharides et lesdits disaccharides étant liés à-O-X-O- par leur carbone anomérique portant initialement le OH hémiacétalique,
∘ l'un au moins des autres OH dudit monosaccharide ou dudit disaccharide étant estérifié par un dérivé lipidique porteur d'au moins une double liaison éventuellement issu d'une huile végétale ou animale ou d'un mélange d'huiles végétales ou animales,
∘ la double liaison ou l'une au moins des doubles liaisons dudit dérivé lipidique étant fonctionnalisée par un groupe choisi parmi les groupes époxy, amines, alcools et acides,
- m correspondant au nombre d'hydroxyles du polyol dont X est issu est un entier compris entre 2 et 10 et
- n, nombre de glucides réducteurs additionnels, est inférieur ou égal à m-2.

3. Dérivés selon l'une quelconque des revendications précédentes **caractérisés en ce que** X est une structure chimique porteuse des groupements hydroxy d'un polyol choisi parmi le glycérol, le xylitol, le phloroglucinol, l'érythritol, le pentaérythritol, le dipentaérythritol, l'arabitol, le ribitol, le sorbitol, le dulcitol, le mannitol, le volemitol, le maltitol, l'isomaltitol et le lactitol, ou d'un diol choisi parmi les diols suivants : 1,3-propanediol, 1,4-butanediol, 1,5-pentanediol, 1,12-dodecanediol, pentaéthylène glycol, 2-butène-1,4-diol, 2-butyne-1,4-diol, 1,4-cyclohexanediol, 2,5-bis(hydroxymethyl)tetrahydrofurane, 1,4-bis(hydroxyméthyl)cyclohexane, isosorbide, 2,5-bis(hydroxymethyl)furane 1,4-bis(hydroxymethyl)benzène, catéchol, résorcinol, hydroquinone, 4,4'-dihydroxybiphenyle et 2,6-dihydroxynaphthalene.

4. Dérivés polyesters d'acides gras de polyglycosides selon l'une quelconque des revendications précédentes, caractérisés en qu'ils répondent à la formule (Ia)
A-O-X-O-Z (Ia)
dans laquelle
- A et Z représentent chacun, indépendamment l'un de l'autre, un glucide réducteur choisi dans le groupe des monosaccharides comprenant le glucose, le fructose, le galactose et le mannose ou dans le groupe des disaccharides comprenant le lactose et le maltose,
∘ lesdits monosaccharides et lesdits disaccharides étant liés à-O-X-O- par le carbone anomérique portant initialement le OH hémiacétalique,
∘ l'un au moins des autres OH dudit monosaccharide ou dudit disaccharide étant estérifié par un dérivé lipidique porteur d'au moins une double liaison éventuellement issu d'une huile végétale ou animale ou d'un mélange d'huiles végétales ou animales,
∘ la double liaison ou l'une au moins des doubles liaisons dudit dérivé lipidique étant fonctionnalisée par un groupe choisi parmi les groupes époxy, amines, alcools et acides et
- X est la structure chimique porteuse des fonctions hydroxy dans un composé choisi dans le groupe comprenant les polyols aliphatiques, cycloaliphatiques, aromatiques.

5. Dérivés selon l'une quelconque des revendications précédentes **caractérisés en ce que** le dérivé lipidique est choisi parmi les acides gras mono- ou polyinsaturés et les esters d'acides gras mono- ou polyinsaturés.

6. Dérivés selon l'une quelconque des revendications précédentes **caractérisés en ce que** A et Z, identiques ou différents, représentent chacun une unité glucosique de formule (II) 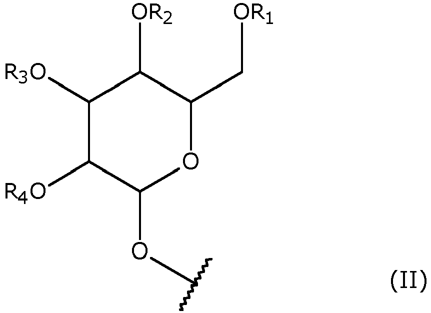 dans laquelle
R₁, R₂, R₃ et R₄, identiques ou représentent chacun :
- soit un atome d'hydrogène,
- soit un groupe -C(O)(C₄-C₃₆)alkyle, avantageusement un groupe -C(O)(C₁₂-C₂₀)alkyle, issu d'un acide gras saturé provenant d'une huile végétale ou animale ou d'un mélange d'huiles végétales ou animales,
- soit un groupe -C(O)(C₄-C₃₆)alcényle, avantageusement un groupe -C(O)(C₁₂-C₂₀)alcényle, issu d'un acide gras insaturé provenant d'une huile végétale ou animale ou d'un mélange d'huiles, végétales ou animales, ledit groupe pouvant porter après fonctionnalisation chimique d'au moins une de ses doubles liaisons, un groupe fonctionnel choisi parmi les fonctions époxy, amines, alcools, et acides, et si plusieurs doubles liaisons sont fonctionnalisées, alors la fonctionnalité est identique pour toutes les doubles liaisons dudit groupe -C(O)(C₄-C₃₆)alcényle,
et à condition que l'un au moins de R₁, R₂, R₃ et R₄ soit un groupe -C(O)(C₄-C₃₆)alcényle fonctionnalisé.

7. Dérivés selon l'une quelconque des revendications précédentes **caractérisé en ce que** X est un résidu de 1,3-propanediol ou un résidu d'hydroquinone.

8. Formulation liquide ou solide comprenant au moins un dérivé selon l'une quelconque des revendications précédentes et éventuellement un agent de réticulation.

9. Résines époxydes biosourcées comprenant le produit de réaction d'un ou de plusieurs dérivés selon l'une quelconque des revendications précédentes, avec au moins un agent de réticulation et éventuellement en présence d'au moins un co-réactif choisi parmi les dérivés glycidyl éthers de polyols époxydés biosourcés.

10. Matériau obtenu par modification chimique ou physique d'une formulation selon la revendication 8.

11. Utilisation d'un matériau selon la revendication 10 comme matrices de composites ou dans la fabrication d'adhésifs, de peintures, de vernis et d'isolants électriques.

## Patentansprüche

1. Fettsäurepolyesterderivate von Polyglycosiden auf Basis eines zwischen 2 und 10 Hydroxy-Funktionen enthaltenden Polyols, wobei die zwei Hydroxy-Funktionen oder mindestens zwei dieser Hydroxy-Funktionen mit dem anomeren Kohlenstoff eines reduzierenden Kohlenhydrats verbunden sind, das für jede Hydroxygruppe gleich oder unterschiedlich ist und aus den Monosacchariden und den Disacchariden ausgewählt ist, in welchen die mindestens eine der weiteren Hydroxygruppen des Monosaccharids oder des Disaccharids durch ein mindestens eine Doppelbindung tragendes Lipidderivat verestert wird, das eventuell aus einem pflanzlichen oder tierischen Öl oder aus einer Mischung aus pflanzlichen oder tierischen Ölen gewonnen wird, wobei die Doppelbindung oder mindestens eine der Doppelbindungen des Lipidderivats durch eine Gruppe funktionalisiert ist, welche aus den Epoxy-, Amin-, Alkohol- und Säuregruppen ausgewählt ist.

2. Fettsäurepolyesterderivate von Polyglycosiden nach Anspruch 1, **dadurch gekennzeichnet, dass** sie der Formel (I) entsprechen 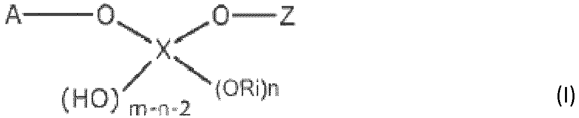 in welcher
- A und Z jeweils unabhängig voneinander ein reduzierendes Kohlenhydrat darstellen, das aus der die Glucose, Fructose, Galactose und Mannose enthaltenden Gruppe der Monosacchariden oder aus der die Laktose und die Maltose enthaltenden Gruppe der Disacchariden ausgewählt ist,
∘ wobei die Monosacchariden und die Disacchariden mit -O-X-O- durch ihren anomeren Kohlenstoff verbunden sind, der ursprünglich das hemiacetalische OH trägt,
∘ wobei das wenigstens eine der weiteren OH des Monosaccharids oder des Disaccharids durch ein mindestens eine Doppelbindung tragendes Lipidderivat verestert ist, das eventuell aus einem pflanzlichen oder tierischen Öl oder aus einer Mischung aus pflanzlichen oder tierischen Ölen gewonnen wird,
∘ wobei die Doppelbindung oder die wenigstens eine der Doppelbindungen des Lipidderivats durch eine Gruppe funktionalisiert ist, die aus den Epoxy-, Amin-, Alkohol- und Säuregruppen ausgewählt ist,
- X eine chemische Struktur darstellt, die die Hydroxy-Funktionen in einer Verbindung trägt, welche aus der die aliphatischen (nicht zyklische Ketten), zykloaliphatischen und aromatischen Polyole enthaltenden Gruppe ausgewählt ist,
- Ri entweder ein Substituent oder mehrere Rₐ bis Rₕ genannte Substituente darstellt, wobei die Rₐ bis Rₕ Substituente, ob gleich oder unterschiedlich, reduzierende Kohlenhydrate sind, die aus der die Glucose, Fructose, Galactose und Mannose enthaltenden Gruppe der Monosacchariden oder aus der die Laktose und Maltose enthaltenden Gruppe der Disacchariden ausgewählt sind,
∘ wobei die Monosacchariden und die Disacchariden mit -O-X-O- durch ihren anomeren Kohlenstoff verbunden sind, der ursprünglich das hemiacetalische OH trägt,
∘ wobei das wenigstens eine der weiteren OH des Monosaccharids oder des Disaccharids durch ein mindestens eine Doppelbindung tragendes Lipidderivat verestert ist, das eventuell aus einem pflanzlichen oder tierischen Öl oder aus einer Mischung aus pflanzlichen oder tierischen Ölen gewonnen wird,
∘ wobei die Doppelbindung oder wenigstens eine der Doppelbindungen des Lipidderivats durch eine Gruppe funktionalisiert ist, die aus den Epoxy-, Amin-, Alkohol- und Säuregruppen ausgewählt ist,
- wobei m für die Hydroxylzahl des Polyols steht, woraus X stammt, und eine Ganzzahl zwischen 2 und 10 ist, und
- n, die Zahl der zusätzlichen reduzierenden Kohlenhydrate, kleiner oder gleich m-2 ist.

3. Derivate nach einem der vorhergehenden Ansprüche, **dadurch gekennzeichnet, dass** X eine chemische Struktur ist, die die Hydroxygruppen eines Polyols trägt, das aus Glycerin, Xylit, Phloroglucin, Erythritol, Pentaerythritol, Dipentaerythritol, Arabit, Ribitol, Sorbitol, Dulcitol, Mannitol, Volemitol, Maltitol, Isomaltitol und Lactitol gewählt oder eines Diols, das aus der folgenden Diole gewählt ist: 1,3-Propandiol, 1,4-Butandiol, 1,5-Pentandiol, 1,12-Dodecandiol, Pentaethylenglycol, 2-Buten-1,4-diol, 2-butin-1,-diol, 1,4-Cyclohexandiol, 2,5-bis(Hydroxymethyl)Tetrahydrofuran, 1,4-bis(Hydroxymethyl)Cyclohexan, Isosorbid, 2,5-bis(Hydroxymethyl)Furan, 1,4-bis(Hydroxymethyl)Benzol, Catechin, Resorcin, Hydrochinon, 4,4'-Dihydroxybiphenyl und 2,6-Dihydroxynaphthalin.

4. Fettsäurepolyesterderivate von Polyglycosiden nach einem der vorhergehenden Ansprüche, **dadurch gekennzeichnet, dass** sie der Formel (la) entsprechen
A-O-X-O-Z (Ia)
in welcher
- A und Z jeweils unabhängig voneinander ein reduzierendes Kohlenhydrat darstellen, das aus der die Glucose, Fructose, Galactose und Mannose enthaltenden Gruppe der Monosacchariden oder aus der die Laktose und die Maltose enthaltenden Gruppe der Disacchariden ausgewählt ist,
∘ wobei die Monosacchariden und die Disacchariden mit -O-X-O- durch ihren anomeren Kohlenstoff verbunden sind, der ursprünglich das hemiacetalische OH trägt,
∘ wobei das wenigstens eine der weiteren OH des Monosaccharids oder des Disaccharids durch ein mindestens eine Doppelbindung tragendes Lipidderivat verestert ist, das eventuell aus einem pflanzlichen oder tierischen Öl oder aus einer Mischung aus pflanzlichen oder tierischen Ölen gewonnen wird,
∘ wobei die Doppelbindung oder wenigstens eine der Doppelbindungen des Lipidderivats durch eine Gruppe funktionalisiert ist, die aus den Epoxy-, Amin-, Alkohol- und Säuregruppen ausgewählt ist,
- X die chemische Struktur ist, die die Hydroxy-Funktionen in einer Verbindung trägt, welche aus der die aliphatischen, zykloaliphatischen und aromatischen Polyole enthaltenden Gruppe ausgewählt ist.

5. Derivate nach einem der vorhergehenden Ansprüche, **dadurch gekennzeichnet, dass** das Lipidderivat aus den einfach oder mehrfach ungesättigten Fettsäuren und den einfach oder mehrfach ungesättigten Fettsäureestern ausgewählt ist.

6. Derivate nach einem der vorhergehenden Ansprüche, **dadurch gekennzeichnet, dass** A und Z, ob gleich oder unterschiedlich, jeweils eine Glucoseeinheit nach der Formel (II) 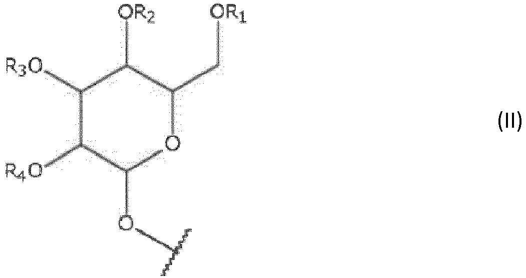 darstellen, in welcher
R₁, R₂, R₃ und R₄, gleich sind oder jeweils darstellen:
- entweder ein Wasserstoffatom,
- oder eine -C(O)(C₄-C₃₆)Alkylgruppe, vorzugsweise eine -C(O)(C₁₂-C₂₀)Alkylgruppe, die aus einer gesättigten Fettsäure aus einem pflanzlichen oder tierischen Öl bzw. aus einer Mischung aus pflanzlichen oder tierischen Ölen gewonnen wird,
- oder eine -C(O)(C₄-C₃₆)Alkenylgruppe, vorzugsweise eine -C(O)(C₁₂-C₂₀)Alkenylgruppe, die aus einer ungesättigten Fettsäure aus einem pflanzlichen oder tierischen Öl oder aus einer Mischung aus pflanzlichen oder tierischen Ölen gewonnen wird, wobei die Gruppe nach chemischer Funktionalisierung mindestens einer seiner Doppelbindungen eine funktionelle Gruppe tragen kann, die aus den Epoxy-, Amin-, Alkohol- und Säurefunktionen ausgewählt ist, und wenn mehrere Doppelbindungen funktionalisiert sind, so ist die Funktion für alle Doppelbindungen der -C(O)(C₄-C₃₆)Alkenylgruppe gleich,
unter der Bedingung, dass mindestens eine von R₁, R₂, R₃ und R₄ eine funktionalisierte - C(O)(C₄-C₃₆)Alkenylgruppe ist.

7. Derivate nach einem der vorhergehenden Ansprüche, **dadurch gekennzeichnet, dass** X ein 1,3-Propandiolrückstand oder ein Hydrochinonrückstand ist.

8. Flüssige oder feste Formulierung mit mindestens einem Derivat nach einem der vorhergehenden Ansprüche und eventuell mit einem Vernetzungsmittel.

9. Biobasierte Epoxidharze, das Reaktionsprodukt aus einem oder mehreren Derivaten nach einem der vorhergehenden Ansprüche enthaltend, mit mindestens einem Vernetzungsmittel und eventuell in Gegenwart mindestens einer co-reaktiven Verbindung, die aus den Glycidyletherderivaten von biobasierten epoxidierten Polyolen ausgewählt ist.

10. Durch chemische oder physische Verarbeitung einer Formulierung nach Anspruch 8 gewonnener Stoff.

11. Verwendung eines Stoffes nach Anspruch 10 als Matrix von Verbundstoffen oder in der Herstellung von Klebstoffen, Farben, Lacken und elektrischen Isolatoren.

## Claims

1. Fatty acid polyester derivatives of polyglycosides formed from a polyol comprising between 2 and 10 hydroxy functions, the 2 hydroxy functions or at least 2 of these hydroxy functions being bound to the anomeric carbon of a reducing carbohydrate that is identical or different for each hydroxy group and selected from the monosaccharides and disaccharides, in which at least one of the other hydroxy groups of said monosaccharide or of said disaccharide is esterified by a lipid derivative bearing at least one double bond optionally originating from a vegetable or animal oil of from a mixture of vegetable or animal oils, the double bond or the at least one of the double bonds of said lipid derivative being functionalized by a group selected from the epoxy, amine, alcohol and acid groups.

2. Fatty acid polyester derivatives of polyglycosides according to claim 1 **characterized in that** they correspond to formula (I) 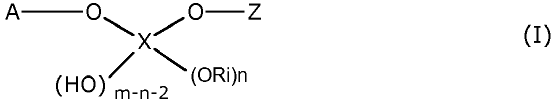 in which
- A and Z each represent, independently of each other, a reducing carbohydrate selected from the group of monosaccharides comprising glucose, fructose, galactose and mannose or from the group of disaccharides comprising lactose and maltose,
∘ said monosaccharides and said disaccharides being bound to -O-X-O- by their anomeric carbon initially bearing the hemiacetal OH,
∘ at least one of the other OH groups of said monosaccharide or said disaccharide being esterified by a lipid derivative bearing at least one double bond optionally originating from a vegetable or animal oil or from a mixture of vegetable or animal oils,
∘ the double bond or the at least one of the double bonds of said lipid derivative being functionalized by a group selected from the epoxy, amine, alcohol and acid groups,
- X represents a chemical structure bearing hydroxy functions in a compound selected from the group comprising the aliphatic (noncyclic chains), cycloaliphatic and aromatic polyols,
- Rᵢ represents either a substituent, or several substituents denoted Rₐ to Rₕ, said substituents Rₐ to Rₕ, identical or different, being reducing carbohydrates selected from the group of monosaccharides comprising glucose, fructose, galactose and mannose or from the group of disaccharides comprising lactose and maltose,
∘ said monosaccharides and said disaccharides being bound to -O-X-O- by their anomeric carbon initially bearing the hemiacetal OH,
∘ at least one of the other OH groups of said monosaccharide or said disaccharide being esterified by a lipid derivative bearing at least one double bond optionally originating from a vegetable or animal oil or from a mixture of vegetable or animal oils,
∘ the double bond or the at least one of the double bonds of said lipid derivative being functionalized by a group selected from the epoxy, amine, alcohol and acid groups,
- m corresponding to the number of hydroxyls of the polyol from which X originated is an integer comprised between 2 and 10 and
- n, the number of additional reducing carbohydrates is less than or equal to m-2.

3. Derivatives according to any one of the preceding claims, **characterized in that** X is a chemical structure bearing hydroxy groups of a polyol selected from glycerol, xylitol, phloroglucinol, erythritol, pentaerythritol, dipentaerythritol, arabitol, ribitol, sorbitol, dulcitol, mannitol, volemitol, maltitol, isomaltitol and lactitol, or a diol selected from the following diols: 1,3-propanediol, 1,4-butanediol, 1,5-pentanediol, 1,12-dodecanediol, pentaethylene glycol, 2-butene-1,4-diol, 2-butyne-1,4-diol, 1,4-cyclohexanediol, 2,5-bis(hydroxymethyl)tetrahydrofuran, 1,4-bis(hydroxymethyl)cyclohexane, isosorbide, 2,5-bis(hydroxymethyl)furan 1,4-bis(hydroxymethyl)benzene, catechol, resorcinol, hydroquinone, 4,4'-dihydroxybiphenyl and 2,6-dihydroxynaphthalene.

4. Fatty acid polyester derivatives of polyglycosides according to any one of the preceding claims **characterized in that** they correspond to formula (Ia)
A-O-X-O-Z (Ia)
in which
- A and Z each represent, independently of each other, a reducing carbohydrate selected from the group of monosaccharides comprising glucose, fructose, galactose and mannose or from the group of disaccharides comprising lactose and maltose,
∘ said monosaccharides and said disaccharides being bound to -O-X-O- by the anomeric carbon initially bearing the hemiacetal OH,
∘ at least one of the other OH groups of said monosaccharide or said disaccharide being esterified by a lipid derivative bearing at least one double bond optionally originating from a vegetable or animal oil or from a mixture of vegetable or animal oils,
∘ the double bond or the at least one of the double bonds of said lipid derivative being functionalized by a group selected from the epoxy, amine, alcohol and acid groups and
- X is the chemical structure bearing hydroxy functions in a compound selected from the group comprising the aliphatic, cycloaliphatic and aromatic polyols,

5. Derivatives according to any one of the preceding claims **characterized in that** the lipid derivative is selected from the mono- or polyunsaturated fatty acids and the mono- or polyunsaturated fatty acid esters.

6. Derivatives according to any one of the preceding claims **characterized in that** A and Z, identical or different, each represent a glucose unit of formula (II) 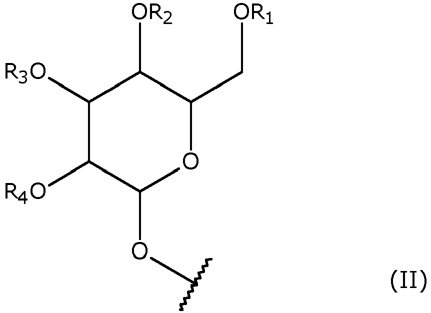 in which
R₁, R₂, R₃ and R₄, identical or different, each represent:
- either a hydrogen atom,
- or a -C(O)(C₄-C₃₆)alkyl group, advantageously a -C(O)(C₁₂-C₂₀)alkyl group, originating from a saturated fatty acid originating from a vegetable or animal oil or from a mixture of vegetable or animal oils,
- or a -C(O)(C₄-C₃₆)alkenyl group, advantageously a -C(O)(C₁₂-C₂₀)alkenyl group, originating from an unsaturated fatty acid originating from a vegetable or animal oil or from a mixture of vegetable or animal oils, said group being able to bear, after chemical functionalization of at least one of its double bonds, a functional group selected from the epoxy, amine, alcohol and acid functions, and if several double bonds are functionalized, then the functionality is identical for all the double bonds of said -C(O)(C₄-C₃₆)alkenyl group,
and provided that at least one of R₁, R₂, R₃ and R₄ is a -C(O)(C₄-C₃₆)alkenyl group functionalized as defined previously.

7. Derivatives according to any one of the preceding claims, **characterized in that** X is a residue of 1,3-propanediol or a residue of hydroquinone.

8. Liquid or solid formulation comprising at least one derivative according to any one of the preceding claims and optionally a cross-linking agent.

9. Biosourced epoxy resins comprising the reaction product of one or more derivatives according to any one of the preceding claims, with at least one cross-linking agent and optionally in the presence of at least one co-reagent selected from the glycidyl ether derivatives of biosourced epoxidized polyols .

10. Material obtained by chemical or physical modification of a formulation according to claim 8.

11. Use of a material according to claim 10 as composite matrices composites or in the manufacture of adhesives, paints, lacquers, and electrical insulation.
